# EUROPÄISCHE PATENTANMELDUNG

(11) **EP 1 005 876 A1**
(43) Veröffentlichungstag der Anmeldung: **07.06.2000**
(21) Anmeldenummer: 99121621.9
(22) Anmeldetag: 30.10.1999
(51) Int. Cl.: A61M 5/32

(54) **Nadelschutzanordnung für Spritzen, Karpulen und dergleichen Injektionsinstrumente**

(30) Priorität: 05.12.1998 DE 19856167
(71) Anmelder: Arzneimittel GmbH Apotheker Vetter & Co. Ravensburg, D-88212 Ravensburg (DE)
(72) Erfinder: Vetter, Helmut, 88212 Ravensburg (DE); Otto, Thomas, 88250 Weingarten (DE); Glocker, Joachim, 88250 Weingarten (DE)
(74) Vertreter: Dziewior, Joachim, Dipl.-Phys. Dr.

(57) **Zusammenfassung**

Die Nadelschutzanordnung ist für Spritzen (2), Karpulen (3) und dergleichen Injektionsinstrumente vorgesehen. Sie weist eine äußere Schutzkappe (7) auf, die die an einem Nadelträger (8) angeordnete Injektionskanüle (9) umschließt und zur Injektion abtrennbar ist. Innerhalb der Schutzkappe (7) ist eine Schutzhülle (10) angeordnet, die fest am Nadelträger (8) angeschlossen ist und die Injektionskanüle (9) Vollständig in sich aufnimmt. Die Schutzhülle (10) ist dabei in axialer Richtung kompressibel bzw. verformbar. Die der Kanülenspitze gegenüberstehende Stirnfläche (10.1) ist so ausgebildet, daß sie einen leichten Durchtritt der Kanüle (9) ermöglicht.

## Beschreibung

Die Erfindung betrifft eine Nadelschutzanordnung für Spritzen, Karpulen und dergleichen Injektionsinstrumente, z. B. auch sogenannte PEN-Systeme, mit einer äußeren Schutzkappe, die die an einem Nadelträger angeordnete Injektionskanüle umschließt und zur Injektion abtrennbar ist.

Derartige Spritzen sind in zahlreichen Ausgestaltungen aus der Praxis bekannt.

In zunehmendem Maße werden bestimmte pharmazeutische Produkte dem Patienten zur Selbstinjektion in die Hand gegeben. Insbesondere bei älteren Patienten besteht dabei die Gefahr einer Verletzung an der Kanüle vor oder nach der Injektion oder auch einer Beschädigung der Kanülenspitze. Bei bestimmten Patienten besteht überdies die Gefahr einer Nadelphobie, der es mit wirksamen Mitteln zu begegnen gilt.

Der Erfindung liegt daher die Aufgabe zugrunde, eine Nadelschutzanordnung der eingangs genannten Art so zu verbessern, daß eine Verletzung bei der Handhabung der Spritze insbesondere durch den Patienten selbst sowie eine eventuelle Nadelphobie weitestgehend vermieden wird.

Eine diese Aufgabe lösende Nadelschutzanordnung ist gekennzeichnet durch eine innerhalb der Schutzkappe angeordnete Schutzhülle, die fest am Nadelträger angeschlossen ist und die Injektionskanüle vollständig in sich aufnimmt, wobei die Schutzhülle in axialer Richtung kompressibel bzw. verformbar ist und die der Kanülenspitze gegenüberstehende Stirnfläche so ausgebildet ist, daß sie einen leichten Durchtritt der Kanüle ermöglicht. Dazu kann die Stirnfläche beispielsweise so dünn ausgebildet sein, daß sie von der Kanüle ohne weiteres durchstochen wird. Es besteht jedoch ebenso die Möglichkeit, eine passende Öffnung in der Stirnfläche für den Durchtritt der Kanüle vorzusehen.

Der durch die Erfindung erreichte Fortschritt besteht im wesentlichen darin, daß die Kanüle vor und nach erfolgter Injektion durch die Schutzhülle umschlossen bleibt, so daß insoweit die Gefahr von Verletzungen bzw. Beschädigungen an der Kanüle erheblich vermindert ist. Darüberhinaus wird der Entstehung einer Nadelphobie entgegengewirkt, zumal die Kanüle auch während der Injektion für den Patienten unsichtbar bleibt. Vorteilhaft ist weiter, daß die Nadelschutzanordnung kostengünstig als steriles Einwegteil hergestellt und gegebenenfalls am Ende des Herstellungs- bzw. Konfektionsablaufs - unter sterilen Bedingungen - dem Primärpackmittel, also z.B. einer vorgefüllten Fertigspritze, als Beilage mit in die Verpackung, also beispielsweise eine Blisterpackung, gegeben werden kann. Da die Nadelschutzanordnung sicher und auf einfache Weise auf dem Primärpackmittel montiert werden kann, läßt sie sich auch von älteren Menschen, die sich bisweilen regelmäßig eine Injektion verabreichen müssen, ohne weiteres handhaben.

In bevorzugter Ausführungsform weist die Schutzhülle eine im wesentlichen zylindrische Gestalt auf, wobei ihre Mantelfläche in der Art eines Faltenbalges gestaltet ist. Diese Gestaltung ermöglicht eine Kompression in axialer Richtung, ohne daß das Auftreten starker radialer Kräfte, die zu einem seitlichen Ausweichen der Schutzhülle führen könnten, zu befürchten ist.

Weiter empfiehlt es sich, daß die Schutzhülle aus elastisch federndem Material besteht, um eine selbsttätige Rückstellung der Schutzhülle beim Zurückziehen der Kanüle nach der Injektion zu erreichen. Zweckmäßigerweise besteht die Schutzhülle dabei aus einem weichelastischen Gummimaterial.

In einer alternativen Ausgestaltung der Erfindung kann in der Wand oder an der Mantelfläche der Schutzhülle ein Federglied in der Art einer Schraubenfeder vorgesehen sein, wodurch ein entsprechend elastisches Verhalten erreicht wird.

Da nach der Montage der Nadelschutzanordnung an der Spritze bzw. Karpule die äußere Schutzkappe abgetrennt werden muß, kann der Nadelträger im Paßsitz in der Schutzkappe angeordnet sein. Es besteht jedoch auch die Möglichkeit, daß die Schutzkappe über eine Sollbruchstelle mit dem Nadelträger verbunden ist.

Um auf einfache Weise eine bestimmte Applikationstiefe für die zu injizierende Substanz vorzugeben, kann im Rahmen der Erfindung ein die Eindringtiefe der Kanüle begrenzendes Anschlagelement vorgesehen sein. Dabei besteht die Möglichkeit, daß die Schutzhülle selbst in komprimiertem Zustand das Anschlagelement bildet, indem sie einen zusammengefalteten Zustand einnimmt, der ein weiteres Zusammenschieben verhindert.

Statt dessen kann der Nadelträger jedoch auch eine vorstehende Ringschulter aufweisen, deren Stirnfläche das Anschlagelement bildet.

Eine weitere zweckmäßige Ausgestaltung besteht darin, daß die zum Aufsetzen auf die Spritze bzw. Karpule eingerichtete Stirnseite der Nadelschutzanordnung vor Gebrauch durch eine lösbare Abdeckung in der Art einer Folie verschlossen ist.

Soweit die Nadelschutzanordnung zur Verwendung an einer Spritze vorgesehen ist, empfiehlt es sich, daß der Nadelträger mit einem Luer Steck- oder Schraubanschluß versehen ist.

Wird die Nadelschutzanordnung dagegen an einer mit einer Bördelkappe versehenen Karpule verwendet, so ist der Nadelträger zweckmäßigerweise mit einer Klemmeinrichtung zum formschlüssigen Aufsetzen auf die Bördelkappe sowie mit einer doppelseitigen Kanüle versehen.

Im folgenden wird die Erfindung an in der Zeichnung dargestellten Ausführungsbeispielen näher erläutert; es zeigen:
- Fig. 1: eine Doppelkammerfertigspritze mit Nadelschutzanordnung, wobei in Teilfig. a) beide Teile für sich im Ausgangszustand dargestellt sind, in Teilfig. b) in Vorbereitung der Injektion die Verschlußkappe von der Spritze entfernt, in Teilfig. c) die Nadelschutzanordnung auf die Spritze aufgesetzt und in Teilfig. d) die Schutzkappe der Nadelschutzanordnung abgenommen ist,
- Fig. 2: die Spritze nach Fig. 1 in nur teilweiser Darstellung bei der Injektion mit maximal eingestochener Kanüle,
- Fig. 3: eine Doppelkammerkarpule mit Nadelschutzanordnung, wobei in Teilfig. a) beide Teile für sich im Ausgangszustand dargestellt sind, in Teilfig. b) die Nadelschutzanordnung auf die Spritze aufgesetzt und in Teilfig. c) die Schutzkappe der Nadelschutzanordnung abgenommen ist,
- Fig. 4: die Karpule nach Fig. 3 in nur teilweiser Darstellung bei der Injektion mit maximal eingestochener Kanüle.

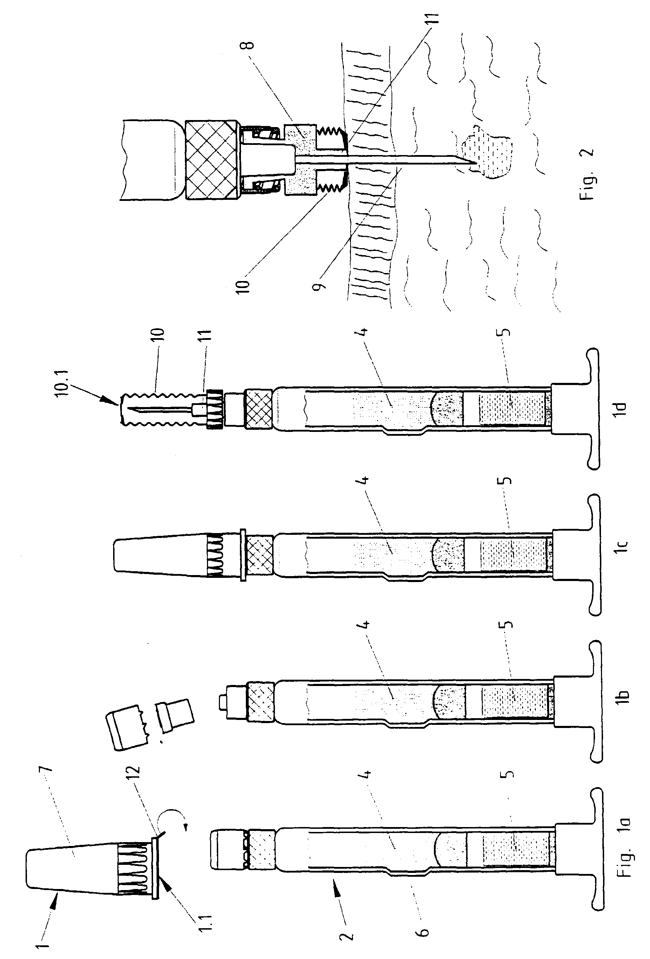

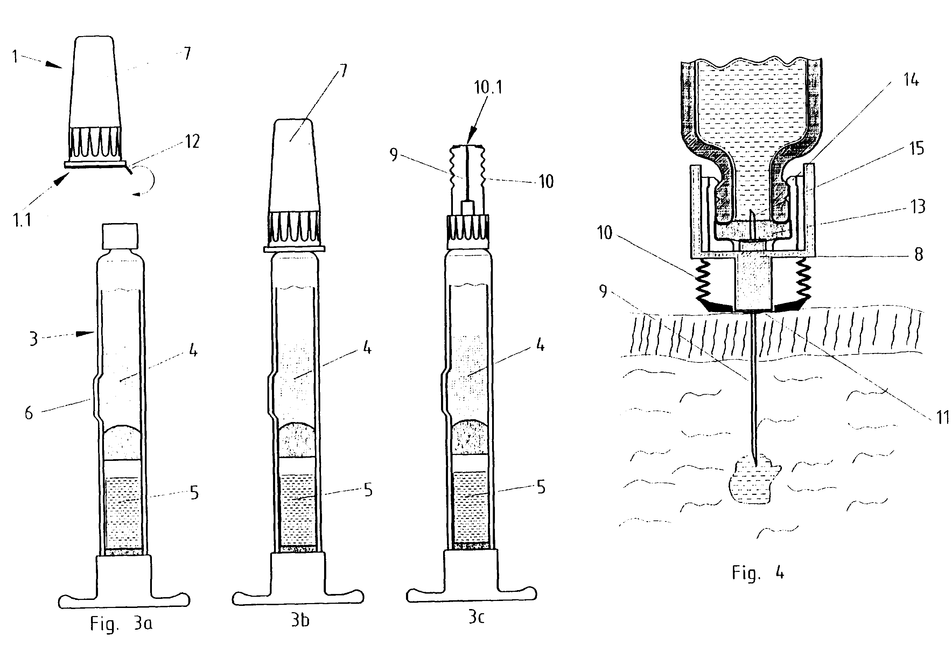

Die in der Zeichnung dargestellte Nadelschutzanordnung 1. ist für Spritzen 2, Karpulen 3 und dergleichen Injektionsinstrumente vorgesehen, wobei in den Fig. 1 und 2 die Anwendung an einer Spritze 2 und in den Figuren 3 und 4 an einer Karpule 3 gezeigt ist. Die Spritze 2 bzw. Karpule 3 ist als Doppelkammerspritze ausgebildet, bei der Wirkstoff 4 und Lösungsmittel 5 bis zur Anwendung getrennt sind und erst unmittelbar vor der Applikation über einen By-pass 6 miteinander gemischt werden. Die Verwendung der Nadelschutzanordnung 1 ist aber keineswegs auf solche Spritzen 2 bzw. Karpulen 3 beschränkt.

Die Nadelschutzanordnung 1 weist zunächst eine äußere Schutzkappe 7 auf, die die an einem Nadelträger 8 angeordnete Injektionskanüle 9 umschließt und die zur Injektion abtrennbar ist.

Da in zunehmendem Maße bestimmte pharmazeutische Produkte dem Patienten zur Selbstinjektion in die Hand gegeben werden und hiervon häufig ältere Patienten betroffen sind, muß Vorsorge getroffen werden, daß die Gefahr einer Verletzung an der Kanüle 9 vor oder nach der Injektion oder auch einer Beschädigung der Kanülenspitze weitestgehend vermieden wird. Auch der bei manchen Patienten bestehenden Gefahr einer Nadelphobie gilt es mit wirksamen Mitteln zu begegnen.

Dazu ist bei der Nadelschutzanordnung 1 innerhalb der Schutzkappe 7 eine Schutzhülle 10 angeordnet, die fest am Nadelträger 8 angeschlossen ist und die Injektionskanüle 9 vollständig in sich aufnimmt. Die Schutzhülle 10 ist in axialer Richtung kompressibel bzw. verformbar ausgebildet, wobei die der Kanülenspitze gegenüberstehende Stirnfläche 10.1 so gestaltet ist, daß sie einen leichten Durchtritt der Kanüle 9 ermöglicht.

Dadurch kann die Kanüle 9 vor und nach erfolgter Injektion durch die Schutzhülle 10 umschlossen bleiben, so daß insoweit die Gefahr von Verletzungen bzw. Beschädigungen an der Kanüle 9 erheblich vermindert ist. Auch wird der Entstehung einer Nadelphobie entgegengewirkt, da die Kanüle 9 auch während der Injektion für den Patienten unsichtbar bleibt. Die Nadelschutzanordnung 1 läßt sich kostengünstig als steriles Einwegteil herstellen und gegebenenfalls am Ende des Herstellungs- bzw. Konfektionsablaufs - unter sterilen Bedingungen - der vorgefüllten Fertigspritze 2 als Beilage mit in die Verpackung beigeben.

Die Schutzhülle 10 weist eine im wesentlichen zylindrische Gestalt auf, wobei ihre Mantelfläche - wie aus den Fig. 1 d) und 3 c) gut zu erkennen - in der Art eines Faltenbalges gestaltet ist. Diese Gestaltung ermöglicht - wie in den Figuren 2 und 4 zu sehen - eine Kompression in axialer Richtung, ohne daß das Auftreten starker radialer Kräfte zu befürchten ist, die zu einem seitlichen Ausweichen der Schutzhülle 10 führen könnten.

Die Schutzhülle 10 besteht aus elastisch federndem Material, um eine selbsttätige Rückstellung der Schutzhülle 10 beim Zurückziehen der Kanüle 9 nach der Injektion zu erreichen. Insbesondere bietet sich hierfür ein weichelastisches Gummimaterial an.

Ebenso besteht die in der Zeichnung nicht dargestellte Möglichkeit, in der Wand oder an der Mantelfläche der Schutzhülle 10 ein Federglied in der Art einer Schraubenfeder vorzusehen, wodurch ein entsprechend elastisches Verhalten erreicht wird.

Da nach der Montage der Nadelschutzanordnung 1 an der Spritze 2 bzw. Karpule 3 die äußere Schutzkappe 7 abgetrennt werden muß, kann der Nadelträger 8 im Paßsitz in der Schutzkappe 7 angeordnet sein, so daß diese nur abzuziehen ist. Es besteht jedoch auch die Möglichkeit, daß die Schutzkappe 7 über eine Sollbruchstelle mit dem Nadelträger 8 verbunden ist.

Um auf einfache Weise eine bestimmte Applikationstiefe für die zu injizierende Substanz vorzugeben, ist ein die Eindringtiefe der Kanüle 9 begrenzendes Anschlagelement vorgesehen. Dazu weist der Nadelträger 8 eine vorstehende Ringschulter 11 auf, deren Stirnfläche das Anschlagelement bildet.

Es besteht jedoch auch die Möglichkeit, daß die Schutzhülle 10 selbst in komprimiertem Zustand das Anschlagelement bildet, indem sie einen zusammengefalteten Zustand einnimmt, der ein weiteres Zusammenschieben verhindert.

Die zum Aufsetzen auf die Spritze 2 bzw. Karpule 3 eingerichtete Stirnseite 1.1 der Nadelschutzanordnung 1 ist vor dem Gebrauch durch eine in den Figuren 1 a) und 3 a) angedeutete lösbare Abdeckung 12 in der Art einer Folie verschlossen.

Soweit die Nadelschutzanordnung 1 zur Verwendung an einer Spritze 2 wie in den Figuren 1 und 2 vorgesehen ist, ist der Nadelträger 8 mit einem Luer Steck- oder gegebenenfalls auch mit einem Schraubanschluß versehen.

Wird die Nadelschutzanordnung 1 dagegen an einer mit einer Bördelkappe 13 versehenen Karpule 3 gemäß den Figuren 3 und 4 verwendet, so ist der Nadelträger 8 zweckmäßigerweise mit einer Klemmeinrichtung 14 zum formschlüssigen Aufsetzen auf die Bördelkappe 13 sowie mit einer doppelseitigen Kanüle 9,15 versehen, wie dies insbesondere aus Fig 4 hervorgeht.

## Patentansprüche

1. Nadelschutzanordnung für Spritzen (2), Karpulen (3) und dergleichen Injektionsinstrumente, mit einer äußeren Schutzkappe (7), die die an einem Nadelträger (8) angeordnete Injektionskanüle (9) umschließt und zur Injektion abtrennbar ist, gekennzeichnet durch eine innerhalb der Schutzkappe (7) angeordnete Schutzhülle (10), die fest am Nadelträger (8) angeschlossen ist und die Injektionskanüle (9) vollständig in sich aufnimmt, wobei die Schutzhülle (10) in axialer Richtung kompressibel bzw. verformbar ist und die der Kanülenspitze gegenüberstehende Stirnfläche (10.1) so ausgebildet ist, daß sie einen leichten Durchtritt der Kanüle (9) ermöglicht.

2. Nadelschutzanordnung nach Anspruch 1, dadurch gekennzeichnet, daß die Schutzhülle (10) eine im wesentlichen zylindrische Gestalt aufweist und ihre Mantelfläche in der Art eines Faltenbalges gestaltet ist.

3. Nadelschutzanordnung nach Anspruch 1 oder 2, dadurch gekennzeichnet, daß die Schutzhülle (10) aus elastisch federndem Material besteht.

4. Nadelschutzanordnung nach einem der Ansprüche 1 bis 3, dadurch gekennzeichnet, daß die Schutzhülle (10) aus einem weichelastischen Gummimaterial besteht.

5. Nadelschutzanordnung nach Anspruch 1 oder 2, dadurch gekennzeichnet, daß in der Wand oder an der Mantelfläche der Schutzhülle (10) ein Federglied in der Art einer Schraubenfeder vorgesehen ist.

6. Nadelschutzanordnung nach einem der Ansprüche 1 bis 5, dadurch gekennzeichnet, daß der Nadelträger (8) im Paßsitz in der Schutzkappe (7) angeordnet ist.

7. Nadelschutzanordnung nach einem der Ansprüche 1 bis 6, dadurch gekennzeichnet, daß die Schutzkappe (7) über eine Sollbruchstelle mit dem Nadelträger (8) verbunden ist.

8. Nadelschutzanordnung nach einem der Ansprüche 1 bis 7, dadurch gekennzeichnet, daß ein die Eindringtiefe der Kanüle (9) begrenzendes Anschlagelement vorgesehen ist.

9. Nadelschutzanordnung nach Anspruch 8, dadurch gekennzeichnet, daß die Schutzhülle (10) selbst in komprimiertem Zustand das Anschlagelement bildet.

10. Nadelschutzanordnung nach Anspruch 8, dadurch gekennzeichnet, daß der Nadelträger (8) eine vorstehende Ringschulter (11) aufweist, deren Stirnfläche das Anschlagelement bildet.

11. Nadelschutzanordnung nach einem der Ansprüche 1 bis 10, dadurch gekennzeichnet, daß die zum Aufsetzen auf die Spritze (2) bzw. Karpule (3) eingerichtete Stirnseite (1.1) vor Gebrauch durch eine lösbare Abdeckung (12) in der Art einer Folie verschlossen ist.

12. Nadelschutzanordnung nach einem der Ansprüche 1 bis 11 zur Verwendung an einer Spritze (2), dadurch gekennzeichnet, daß der Nadelträger (8) mit einem Luer Steck- oder Schraubanschluß versehen ist.

13. Nadelschutzanordnung nach einem der Ansprüche 1 bis 11 zur Verwendung an einer mit einer Bördelkappe (13) versehenen Karpule (3), dadurch gekennzeichnet, daß der Nadelträger (8) mit einer Klemmeinrichtung (14) zum formschlüssigen Aufsetzen auf die Bördelkappe (13) sowie mit einer doppelseitigen Kanüle (9,15) versehen ist.
